# EUROPÄISCHE PATENTANMELDUNG

(11) **EP 4 850 644 A1**
(43) Veröffentlichungstag der Anmeldung: **09.12.2026**
(21) Anmeldenummer: 26175607.6
(22) Anmeldetag: 30.04.2026
(51) Int. Cl.: A61F 9/007

(54) **OPHTHALMOLOGISCHE KASSETTE, SYSTEM UND VERFAHREN ZUM ERSETZEN EINER OPHTHALMOLOGISCHEN KASSETTE**

(30) Priorität: 03.06.2025 DE 102025121612
(71) Anmelder: Geuder AG, 69126 Heidelberg (DE)
(72) Erfinder: Stegmaier, Lukas, 69226 Nußloch (DE); Prisching, Thomas, 68782 Brühl (DE)
(74) Vertreter: Ullrich & Naumann PartG mbB

(57) **Zusammenfassung**

Die Erfindung betrifft eine ophthalmologische Kassette, insbesondere für ein Phakoemulsifikationssystem, mit einem Gehäuse, einer Aspirationsleitung, einer Irrigationsleitung und einem Anschlag für eine Rollenpumpe, wobei das Gehäuse zumindest ein Aspirationsgehäuse und zumindest ein Irrigationsgehäuse umfasst, wobei die Aspirationsleitung in dem Aspirationsgehäuse angeordnet ist und wobei die Irrigationsleitung in dem Irrigationsgehäuse angeordnet ist, wobei das Aspirationsgehäuse und das Irrigationsgehäuse aneinander angeordnet sind.

## Beschreibung

Die Erfindung betrifft eine ophthalmologische Kassette, insbesondere für eine ophthalmologische Konsole, mit einem Gehäuse, einer Aspirationsleitung, einer Irrigationsleitung und einem Anschlag für eine Rollenpumpe.

Die Erfindung betrifft weiter ein ophthalmologisches System, umfassend eine ophthalmologische Konsole mit einem Schacht und eine ophthalmologische Kassette.

Darüber hinaus betrifft die Erfindung ein Verfahren zum Ersetzen einer ophthalmologischen Kassette.

Ophthalmologische Systeme der gattungsbildenden Art sind in den unterschiedlichsten Ausführungen aus der Praxis bekannt. Dabei kann es sich beispielsweise um ein Phakoemulsifikationssystem handeln. Ein solches besteht aus einer ophthalmologischen Konsole, an die ein Handstück anschließbar ist. Die ophthalmologische Konsole kann beispielsweise eine Phakoemulsifikationskonsole sein. Über die Konsole wird insbesondere eine Irrigationsflüssigkeit, Unterdruck sowie elektrische Leistung zu Verfügung gestellt. Im Rahmen einer Kataraktoperation, bei der die getrübte Linse eines Patienten aus dem Auge zu entfernen ist, wird eine von dem Handstück getragene Nadel in das Auge eingeführt und mit einer Ultraschallschwingung beaufschlagt. Durch die Schwingung wird die Linse zerstört bzw. emulsifiziert. Die Linsentrümmer werden durch die Nadel und das Handstück aus dem Auge abgesaugt - aspiriert - wozu von der Konsole ein Unterdruck erzeugt wird. Um das Auge vor Verletzungen zu schützen, muss der Kammerdruck während des Eingriffs aufrecht erhalten werden. Hierzu wird dem Auge über die Konsole und das Handstück eine Irrigationsflüssigkeit zugeleitet, wodurch der Kammerdruck gezielt auf dem notwendigen Niveau gehalten wird. Zum Einbringen von Irrigationsflüssigkeit und zum Absaugen und Sammeln der Linsentrümmer sowie der Irrigationsflüssigkeit ist es bekannt, Kassetten vorzusehen, die in eine entsprechende Aufnahme an der Konsole einbringbar sind.

Ophthalmologische Kassette der in Rede stehenden Art sind seit Jahren aus der Praxis bekannt. Diese Kassetten umfassen eine Irrigationsleitung durch die Irrigationsflüssigkeit zu den Handstücken der Konsole geleitet werden kann, sowie eine Aspirationsleitung, die Aspirationsflüssigkeit von den Handstücken der Konsole zu einem Aspirationsbehälter leiten kann. Die Aspirations- und die Irrigationsleitungen bestehen aus einem Schlauch, der durch eine in der Konsole angeordnete Rollenpumpe gewalkt werden kann, um so Fluid durch den Schlauch zu fördern.

Um die Gefahr einer Kontamination verschiedener Patienten zu verhindern ist es bekannt geworden, nach jeder Operation die Kassette zu wechseln.

Ein Nachteil davon ist, dass dies viel Abfall produziert, da die Kassetten Einwegprodukte sind. Außerdem ist die Produktion von Kassetten mit hohen Kosten verbunden. Es ist ebenfalls bekannt geworden, statt der gesamten Kassette lediglich das Schlauchsystem innerhalb der Kassetten auszuwechseln. Dies ist jedoch mit einem hohen Zeitaufwand verbunden.

Der vorliegenden Erfindung liegt daher die Aufgabe zugrunde, eine ophthalmologische Kassette, ein ophthalmologisches System und ein Verfahren zum Ersetzen einer Kassette der eingangs genannten Art derart auszugestalten und weiterzubilden, dass der Bedarf an Wegwerfprodukten reduziert wird, die Kosten gesenkt werden und die benötigte Zeit zum Wechseln einer Kassette minimiert wird.

Erfindungsgemäß wird die voranstehende Aufgabe durch die Merkmale des Anspruches 1 gelöst. Danach ist eine ophthalmologische Kassette vorgesehen, insbesondere für ein Phakoemulsifikationssystem, mit einem Gehäuse, einer Aspirationsleitung, einer Irrigationsleitung und einem Anschlag für eine Rollenpumpe.

Die Kassette ist dadurch gekennzeichnet, dass das Gehäuse zumindest ein Aspirationsgehäuse und zumindest ein Irrigationsgehäuse umfasst, wobei die Aspirationsleitung in dem Aspirationsgehäuse angeordnet ist und wobei die Irrigationsleitung in dem Irrigationsgehäuse angeordnet ist, wobei das Aspirationsgehäuse und das Irrigationsgehäuse aneinander angeordnet sind.

Darüber hinaus wird die voranstehende Aufgabe durch die Merkmale des Anspruchs 6 gelöst. Danach ist ein ophthalmologisches System, umfassend eine ophthalmologische Konsole mit einer Aufnahme und eine ophthalmologische Kassette gemäß einem der Ansprüche 1 bis 5, wobei die ophthalmologische Kassette in der Aufnahme der Konsole anordenbar ist.

Die Aufnahme kann insbesondere als ein Schacht ausgebildet sein, in den die Kassette einführbar und/oder einsetzbar ist.

Außerdem wird die voranstehende Aufgabe durch die Merkmale des Anspruchs 10 gelöst. Danach ist ein Verfahren zum Ersetzen einer ophthalmologischen Kassette gemäß einem der Ansprüche 1 bis 5 vorgesehen, insbesondere nach Nutzung in einem ophthalmologischen System gemäß einem der Ansprüche 6 bis 9, umfassend die Schritte:
- Entfernen des Aspirationsgehäuses von der ophthalmologischen Kassette;
- Anordnen eines neuen Aspirationsgehäuses an die ophthalmologische Kassette

Der Begriff "neues Aspirationsgehäuse" ist im weitesten Sinne zu verstehen und bezieht sich in der Beschreibung, vorzugsweise in den Ansprüchen auf ein anderes Aspirationsgehäuse, das nicht dem Aspirationsgehäuse entspricht, das zuvor entfernt worden ist, oder auf das gleiche Aspirationsgehäuse, wobei jedoch das Schlauchsystem des Aspirationsgehäuses ausgetauscht worden ist.

Der Begriff "aneinander angeordnet" ist im Weitesten Sinne zu verstehen und bezieht sich in der Beschreibung, vorzugsweise in den Ansprüchen darauf, dass das Aspirationsgehäuse und das Irrigationsgehäuse nebeneinander angeordnet sind. Dabei können das Aspirationsgehäuse und das Irrigationsgehäuse direkt, das heißt ohne Spalt, aneinander angeordnet sein und/oder das Aspirationsgehäuse und das Irrigationsgehäuse können mit einem Spalt zwischen dem Irrigationsgehäuse und dem Aspirationsgehäuse aneinander angeordnet sein. Die Breite des Spalts kann insbesondere zwischen 0,5 mm und 100 mm betragen, insbesondere zwischen 1 und 10 mm. Mit anderen Worten können das Aspirationsgehäuse und das Irrigationsgehäuse miteinander in Kontakt stehen und/oder durch einen Spalt getrennt sein.

In erfindungsgemäßer Weise ist zunächst erkannt worden, dass durch das Anordnen des Aspirationsgehäuses an dem Irrigationsgehäuse die Herstellungskosten gesenkt werden können, weil beispielsweise das Aspirationsgehäuse und das Irrigationsgehäuse getrennt hergestellt werden können. Anschließend können das Aspirations- und das Irrigationsgehäuse aneinander angeordnet werden und so eine Kassette bilden. Dabei ist es denkbar, dass das Aspirationsgehäuse und das Irrigationsgehäuse fest miteinander verbunden werden, beispielsweise verklebt werden oder lösbar aneinander festgelegt sind.

Gemäß einer vorteilhaften Weiterbildung der Erfindung sind das Aspirationsgehäuse und das Irrigationsgehäuse identisch ausgebildet. Mit anderen Worten sind das das Aspirationsgehäuse und das Irrigationsgehäuse gleich aufgebaut. Das heißt, dass eine Kassette durch Aneinanderfügen von zwei Gehäusehälften gebildet werden kann, wobei die Gehäusehälften identisch sind. Ein Vorteil hiervon ist, dass die Herstellungskosten vereinfacht werden, weil nicht zwei verschiedene Gehäusehälften hergestellt werden müssen.

Vorteilhafterweise sind die Aspirations- und/oder Irrigationsleitung jeweils lösbar von dem Aspirations- und/oder Irrigationsgehäuse ausgebildet. Somit kann nach dem Entfernen des Aspirationsgehäuses und/oder des Irrigationsgehäuses das Schlauchsystem auf einfache Weise ausgetauscht werden. Anschließend können das Aspirationsgehäuses und/oder des Irrigationsgehäuses wiederverwendet werden. Insbesondere kann lediglich das Schlauchsystem des Aspirationsgehäuses ausgetauscht werden, da das Irrigationssystem nicht nach jeder Operation ausgetauscht werden muss.

Es ist vorteilhaft, wenn das Aspirationsgehäuse und das Irrigationsgehäuse durch eine formschlüssige Verbindung, insbesondere eine Nut-Feder Verbindung, eine Bügelklammer und/oder eine Schwalbenschwanz-Verbindung, aneinander festgelegt sind. Ein Vorteil hiervon ist, dass die ophthalmologische Kassette eine höhere mechanische Stabilität aufweist.

Gemäß einer vorteilhaften Weiterbildung der Erfindung sind das Aspirationsgehäuse und das Irrigationsgehäuse lösbar aneinander angeordnet. Bei der Irrigation gelangt keine Flüssigkeit aus dem Auge in das Irrigationsgehäuse, sodass dieses nicht nach jeder Operation ausgetauscht werden braucht. Um dies zu ermöglichen, kann die ophthalmologische Kassette aus einem Irrigationsgehäuse und einem Aspirationsgehäuse gebildet sein, wobei das Irrigationsgehäuse und das Aspirationsgehäuse voneinander trennbar sind. Somit kann nach der Operation das Aspirationsgehäuse von der Kassette entfernt und ersetzt werden, wohingegen das Irrigationsgehäuse weiter genutzt werden kann. Auf diese Weise kann der Verbrauch an Einwegprodukten halbiert werden. Es ist denkbar, dass das Aspirationsgehäuse und das Irrigationsgehäuse lediglich aneinander angelegt sind. Es ist ebenfalls denkbar, dass das Aspirationsgehäuse und das Irrigationsgehäuse durch eine lösbare Verbindung miteinander verbunden sind, beispielsweise eine lösbare Klebeverbindung oder eine lösbare formschlüssige Verbindung.

In einer bevorzugten Weiterbildung der Erfindung ist in der Aufnahme eine Festlegeeinrichtung angeordnet, die dazu ausgebildet ist, das Irrigationsgehäuse festzulegen. Mittels der Festlegeeinrichtung kann das Irrigationsgehäuse der ophthalmologischen Kassette in der Aufnahme, insbesondere dem Schacht der Konsole, festgelegt werden. Somit das Aspirationsgehäuse aus dem Schacht entnommen werden kann, wohingegen das Irrigationsgehäuse in dem Schacht verbleibt. Ein Vorteil hiervon ist, dass der Austausch des Aspirationsgehäuses schneller erfolgen kann.

Es ist ebenfalls denkbar, dass in der Aufnahme eine Festlegeeinrichtung angeordnet ist, die dazu ausgebildet ist, das Aspirationsgehäuse festzulegen. Mittels der Festlegeeinrichtung kann das Aspirationsgehäuse der ophthalmologischen Kassette in der Aufnahme, vorzugsweise in dem Schacht, der Konsole festgelegt werden. Somit das Irrigationsgehäuse aus dem Schacht entnommen werden kann, wohingegen das Aspirationsgehäuse in dem Schacht verbleibt. Ein Vorteil hiervon ist, dass der Austausch des Irrigationsgehäuses schneller erfolgen kann.

Es ist denkbar, dass das Irrigationsgehäuse und das Aspirationsgehäuse innerhalb des Schachts voneinander lösbar sind, wenn die ophthalmologische Kassette in dem Schacht angeordnet ist. Beispielsweise könnte das Irrigationsgehäuse lediglich an dem Aspirationsgehäuse angelegt sein, sodass das Aspirationsgehäuse aus der Aufnahme der Konsole entnehmbar ist, während die Kassette in der Konsole angeordnet ist. Es ist ebenfalls denkbar, dass eine Verbindung zwischen dem Irrigationsgehäuse und dem Aspirationsgehäuse lösbar ist, währen die Kassette in der Konsole eingesetzt ist, sodass anschließend das Aspirationsgehäuse entfernt werden kann. Ein Vorteil hiervon ist, dass das Aspirationsgehäuse auf einfache Weise entfernt werden kann.

Gemäß einer vorteilhaften Weiterbildung der Erfindung wird zunächst die ophthalmologische Kassette aus der ophthalmologischen Konsole entfernt. Das heißt, dass zunächst die gesamte Kassette aus der Konsole entnommen wird und anschließend das Aspirationsgehäuse von der Kassette entfernt wird, sodass ein neues Aspirationsgehäuse angeordnet werden kann. Ein Vorteil hiervon ist, dass das Aspirationsgehäuse leichter ausgetauscht werden kann.

Es gibt nun verschiedene Möglichkeiten, die Lehre der vorliegenden Erfindung in vorteilhafter Weise auszugestalten und weiterzubilden. Dazu ist einerseits auf die dem Anspruch 1 nachgeordneten Ansprüche und andererseits auf die nachfolgende Erläuterung bevorzugter Ausführungsbeispiele der Erfindung anhand der Zeichnung zu verweisen. In Verbindung mit der Erläuterung der bevorzugten Ausführungsbeispiele der Erfindung anhand der Zeichnung werden auch im Allgemeinen bevorzugte Ausgestaltungen und Weiterbildungen der Lehre erläutert. In der Zeichnung zeigen
- Fig. 1: eine ophthalmologische Kassette gemäß einer Ausführungsform der vorliegenden Erfindung,
- Fig. 2: eine Aspirationsgehäuse gemäß einer Ausführungsform der vorliegenden Erfindung,
- Fig. 3: ein ophthalmologisches System gemäß einer Ausführungsform der vorliegenden Erfindung,
- Fig. 4: Schritte eines Verfahrens gemäß einer Ausführungsform der vorliegenden Erfindung.

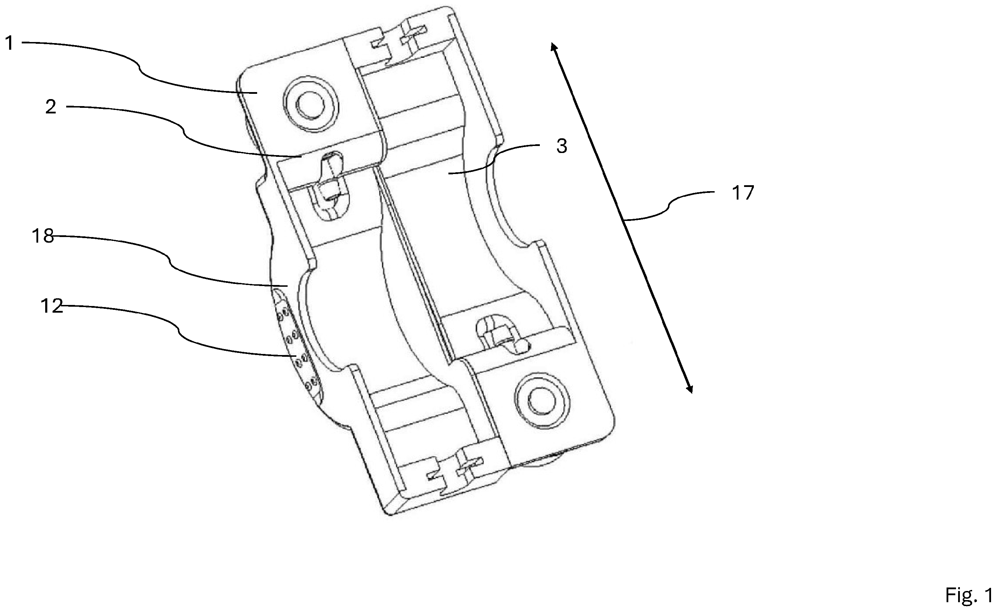

Fig. 1 zeigt eine ophthalmologische Kassette gemäß einer Ausführungsform der vorliegenden Erfindung.

Die ophthalmologische Kassette 1 besteht aus zwei Gehäusehälften, einem Irrigationsgehäuse 2 und einem Aspirationsgehäuse 3. Das Irrigationsgehäuse 2 und das Aspirationsgehäuse 3 sind identisch aufgebaut und lediglich gedreht zueinander aneinander angeordnet. Deswegen wird im Folgenden lediglich das Aspirationsgehäuse 3 beschrieben, alle Ausführungen gelten jedoch gleichermaßen für das Irrigationsgehäuse 2. Zusammen bilden das Irrigationsgehäuse 2 und das Aspirationsgehäuse 3 das Gehäuse 18 der ophthalmologischen Kassette 1.

Das Irrigationsgehäuse 2 und das Aspirationsgehäuse 3 sind aneinander angeordnet. Es ist denkbar, dass das Irrigationsgehäuse 2 und das Aspirationsgehäuse 3 aneinander festgelegt sind, beispielsweise durch eine formschlüssige Verbindung. Es ist ebenfalls denkbar, dass das Irrigationsgehäuse 2 und das Aspirationsgehäuse 3 lediglich lose aneinander anliegen und erst durch die Form eines Schachts der Konsole relativ zueinander fest positioniert werden. Darüber hinaus ist auch denkbar, dass das Irrigationsgehäuse 2 und das Aspirationsgehäuse 3 zusammen in einem gemeinsamen Gehäuse der Kassette angeordnet sind und dadurch relativ zueinander positioniert werden. In diesem Fall sind das Irrigationsgehäuse 2 und/oder das Aspirationsgehäuse 3 lösbar von dem gemeinsamen Gehäuse der Kassette.

In Figur 1 sind das Irrigationsgehäuse 2 und das Aspirationsgehäuse 3 identisch ausgebildet. Es ist ebenfalls denkbar, dass das Irrigationsgehäuse 2 und das Aspirationsgehäuse 3 lediglich im Wesentlichen identisch sind. Das heißt, dass die geometrische Form des Irrigationsgehäuses 2 zumindest zu 80%, insbesondere zumindest zu 90%, vorzugsweise zumindest zu 95%, mit der geometrischen Form des Aspirationsgehäuses 3 übereinstimmt.

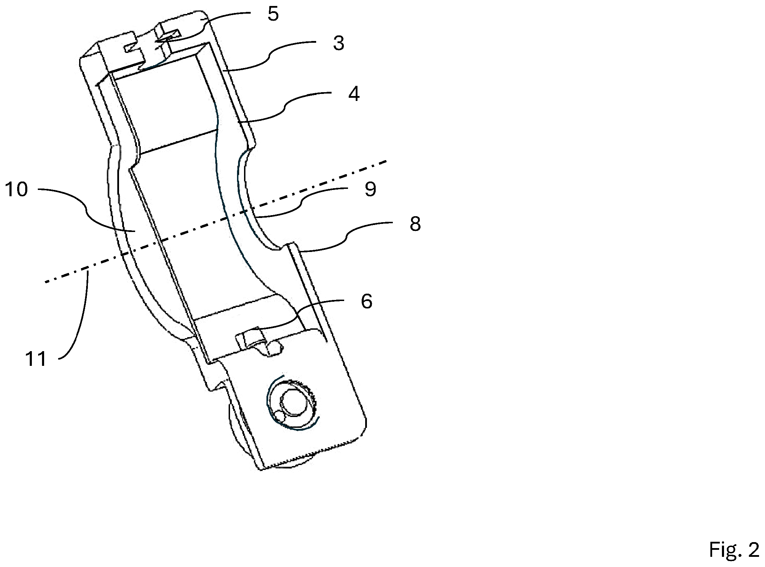

Das Aspirationsgehäuse ist in Figur 2 gezeigt. Es umfasst eine äußere Wandung 4, in dem ein Anschluss 5 vorgesehen ist, in dem ein Schlauch (nicht gezeigt) einer Aspirationsleitung angeordnet werden kann. Die Aspirationsleitung kann entlang des Aspirationsgehäuses bis zu einem Auslass 6 verlaufen. Zwischen dem Anschluss 5 und dem Auslass 6 ist ein Anschlag 7 in Form eines Bogens vorgesehen. Der Anschlag 7 ist dazu ausgebildet, an eine Rollenpumpe angeordnet zu werden, sodass ein auf den Anschlag 7 angeordneter Schlauch der Aspriationsleitung durch die Rollenpumpe gewalkt werden kann.

An einer ersten Seite 8 der Wandung 4 ist eine kreisförmige Aussparung 9 vorgesehen, in der die Rollenpumpe und/oder eine Achse der Rollenpumpe eingesetzt werden kann. Auf der der ersten Seite 8 gegenüberliegenden zweiten Seite 10 der Wandung 4 ist die Wandung 4 durchgängig ausgebildet. An dieser zweiten Seite 10 können das Irrigationsgehäuse 2 und das Aspirationsgehäuse 3 aneinander angeordnet werden.

Das Aspirationsgehäuse 3 ist im Querschnitt gesehen achsensymmetrisch zu einer Mittelachse 11, sodass zwei Aspirationsgehäuse aneinander anordenbar sind, selbst wenn eines Aspirationsgehäuse um 180° gedreht ist.

In Figur 1 ist dargestellt, dass die Anschläge 7, 7' des Aspirationsgehäuses 3 und des Irrigationsgehäuses 2 versetzt sind relativ zu einer Haupterstreckungsrichtung 17 der Kassette. Das heißt, dass zwei verschiedene Rollenpumpen dazu genutzt werden können, Fluid durch die Aspirations- und Irrigationsleitung (nicht gezeigt) zu pumpen. Es ist ebenfalls denkbar, dass die Anschläge 7, 7' auf derselben Höhe relativ zu der Haupterstreckungsrichtung 17 angeordnet sind, sodass sowohl die Aspirations- als auch die Irrigationsleitung durch eine gemeinsame Rollenpumpe walkbar sind. In diesem Fall könnte eine zusätzliche Aussparung in der zweiten Seite 10 angeordnet sein.

Unterhalb der kreisförmigen Ansparung 9 ist eine Festlegeeinrichtung 12, hier in Form von mehreren Öffnungen, vorgesehen (lediglich in Figur 1 sichtbar). Mittels dieser Festlegeeinrichtung 12 kann das Aspirationsgehäuse 3 in einem Schacht einer Konsole festgelegt werden.

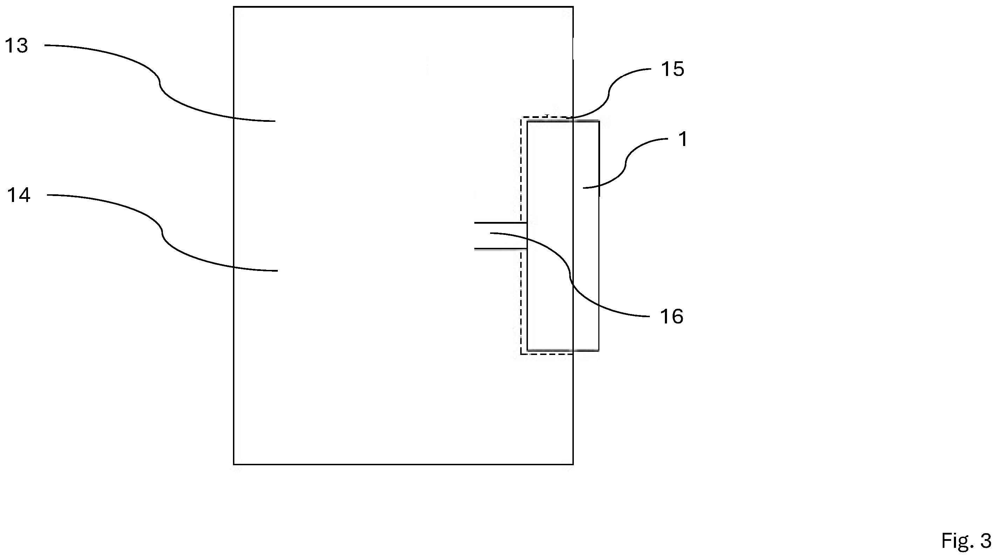

Eine solche Konsole ist in Figur 3 gezeigt.

Die Kassette 1 ist in einem Schacht 15 der Konsole 13 angeordnet. Konsole 13 und Kassette 1 bilden zusammen ein ophthalmologisches System 14. In der Konsole 13 ist ein Stempel 16 vorgesehen, der in die Festlegeeinrichtung (nicht gezeigt in Fig. 3) der Kassette 1 eingreifen und diese festlegen kann. Es ist denkbar, dass zwei Stempel vorgesehen sind, die jeweils in das Aspirations- und Irrigationsgehäuse eingreifen können.

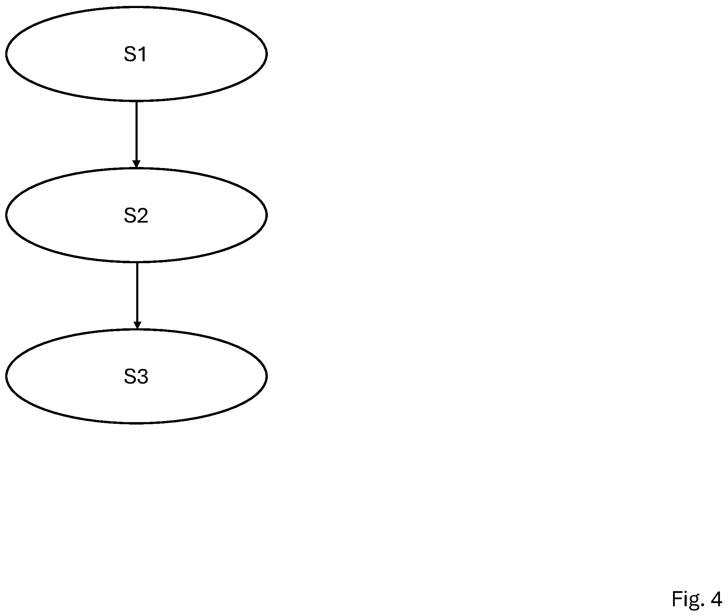

Figur 4 zeigt in schematischer Form Schritte eines Verfahrens gemäß einer Ausführungsform der vorliegenden Erfindung.

Das Verfahren gemäß Figur 4 ist insbesondere dazu geeignet, eine Kassette nach einer erfolgten Operation wieder in einen gebrauchsfertigen Zustand zu überführen, um eine weitere Operation durchführen zu können.

In einem optionalen Schritt S1 wird zunächst die Kassette aus dem Schacht entfernt. Anschließend wird in einem Schritt S2 das Aspirationsgehäuse aus von der Kassette entfernt. Danach wird ein neues Aspirationsgehäuse in einem weiteren Schritt S3 an der Kassette angeordnet. Es ist ebenfalls denkbar, dass lediglich das Schlauchsystem aus dem Aspirationsgehäuse gegen ein neues Schlauchsystem ausgetauscht wird. Das heißt, dass die Aspirationsleitung des Aspirationsgehäuses ausgetauscht wird und anschließend das Aspirationsgehäuse wieder an der Kassette angeordnet wird.

Das eingangs beschriebene Verfahren ist ebenfalls dazu geeignet, stattdessen das Irrigationsgehäuse auszutauschen.

Hinsichtlich weiterer vorteilhafter Ausgestaltungen der erfindungsgemäßen Vorrichtung wird zur Vermeidung von Wiederholungen auf den allgemeinen Teil der Beschreibung sowie auf die beigefügten Ansprüche verwiesen.

Schließlich sei ausdrücklich darauf hingewiesen, dass die voranstehend beschriebenen Ausführungsbeispiele der erfindungsgemäßen Vorrichtung lediglich zur Erörterung der beanspruchten Lehre dienen, diese jedoch nicht auf die Ausführungsbeispiele einschränken.

### Bezugszeichenliste

- 1: Kassette
- 2: Irrigationsgehäuse
- 3: Aspirationsgehäuse
- 4: Wandung
- 5: Anschluss
- 6: Auslass
- 7: Anschlag
- 8: Erste Seite
- 9: Aussparung
- 10: Zweite Seite
- 11: Mittlachse
- 12: Festlegeinrichtung
- 13: Konsole
- 14: System
- 15: Schacht
- 16: Stempel
- 17: Haupterstreckungsrichtung
- 18: Gehäuse

## Patentansprüche

1. Ophthalmologische Kassette (1), insbesondere für ein Phakoemulsifikationssystem, mit einem Gehäuse (18), einer Aspirationsleitung, einer Irrigationsleitung und einem Anschlag (7) für eine Rollenpumpe,
**dadurch gekennzeichnet, dass** das Gehäuse (18) zumindest ein Aspirationsgehäuse (3) und zumindest ein Irrigationsgehäuse (2) umfasst, wobei die Aspirationsleitung in dem Aspirationsgehäuse (3) angeordnet ist und wobei die Irrigationsleitung in dem Irrigationsgehäuse (2) angeordnet ist, wobei das Aspirationsgehäuse (3) und das Irrigationsgehäuse (2) aneinander angeordnet sind.

2. Ophthalmologische Kassette (1) gemäß Anspruch 1, **dadurch gekennzeichnet, dass** das Aspirationsgehäuse (3) und das Irrigationsgehäuse (2) identisch ausgebildet sind.

3. Ophthalmologische Kassette (1) gemäß Anspruch 1 oder 2, **dadurch gekennzeichnet, dass** die Aspirations- und/oder Irrigationsleitung jeweils lösbar von dem Aspirations- und/oder Irrigationsgehäuse (2) ausgebildet sind.

4. Ophthalmologische Kassette (1) gemäß einem der Ansprüche 1 bis 3, **dadurch gekennzeichnet, dass** das Aspirationsgehäuse (3) und das Irrigationsgehäuse (2) durch eine formschlüssige Verbindung, insbesondere eine Nut-Feder Verbindung, eine Bügelklammer und/oder eine Schwalbenschwanz-Verbindung, aneinander festgelegt sind.

5. Ophthalmologische Kassette (1) gemäß einem der Ansprüche 1 bis 4, **dadurch gekennzeichnet, dass** das das Aspirationsgehäuse (3) und das Irrigationsgehäuse (2) lösbar aneinander angeordnet sind.

6. Ophthalmologisches System (14), umfassend eine ophthalmologische Konsole (13) mit einer Aufnahme (15) und eine ophthalmologische Kassette (1) gemäß einem der Ansprüche 1 bis 5, wobei die ophthalmologische Kassette (1) in der Aufnahme (15) der Konsole (13) anordenbar ist.

7. Ophthalmologisches System (14) gemäß Anspruch 6, **dadurch gekennzeichnet, dass** in der Aufnahme (15) eine Festlegeeinrichtung (12) angeordnet ist, die dazu ausgebildet ist, das Irrigationsgehäuse (2) festzulegen.

8. Ophthalmologisches System (14) gemäß Anspruch 6 oder 7, **dadurch gekennzeichnet, dass** in der Aufnahme (15) eine Festlegeeinrichtung (12) angeordnet ist, die dazu ausgebildet ist, das Aspirationsgehäuse (3) festzulegen.

9. Ophthalmologisches System (14) gemäß einem der Ansprüche 6 bis 8, **dadurch gekennzeichnet, dass** das Irrigationsgehäuse (2) und das Aspirationsgehäuse (3) innerhalb der Aufnahme (15) voneinander lösbar sind, wenn die ophthalmologische Kassette (1) in dem Schacht (15) angeordnet ist.

10. Verfahren zum Ersetzen einer ophthalmologischen Kassette (1) gemäß einem der Ansprüche 1 bis 5, insbesondere nach Nutzung in einem ophthalmologischen System (14) gemäß einem der Ansprüche 6 bis 9, umfassend die Schritte:
∘ Entfernen (S2) des Aspirationsgehäuses (3) von der ophthalmologischen Kassette (1);
∘ Anordnen (S3) eines neuen Aspirationsgehäuses (3) an die ophthalmologische Kassette (1)

11. Verfahren gemäß Anspruch 10, wobei zunächst die ophthalmologische Kassette (1) aus der ophthalmologischen Konsole (13) entfernt wird (S1).
